# EUROPÄISCHE PATENTSCHRIFT

(11) **EP 1 667 612 B1**
(45) Veröffentlichungstag und Bekanntmachung des Hinweises auf die Patenterteilung: **31.12.2008**
(21) Anmeldenummer: 05730084.0
(22) Anmeldetag: 06.04.2005
(51) Int. Cl.: A61F 2/16

(54) **FOKUSSIONSFÄHIGE KÜNSTLICHE LINSE FÜR EIN AUGE**
FOCUSABLE ARTIFICIAL LENS FOR AN EYE
LENTILLE ARTIFICIELLE APTE A LA FOCALISATION POUR UN OEIL

(30) Priorität: 07.04.2004 DE 102004017283
(43) Veröffentlichungstag der Anmeldung: 14.06.2006
(73) Patentinhaber: Carl Zeiss Surgical GmbH, 73447 Oberkochen (DE)
(72) Erfinder: OBREBSKI, Andreas, 40489 Düsseldorf (DE)
(74) Vertreter: Müller, Thomas
(86) Internationale Anmeldenummer: PCT/EP2005/003618
(87) Internationale Veröffentlichungsnummer: WO 2005/096999

(56) Entgegenhaltungen:
- EP-A- 0 356 050
- US-A- 4 816 031
- US-A- 5 443 506
- US-A- 6 117 171

## Beschreibung

Die vorliegende Erfindung betrifft eine künstliche Linse für ein Auge, insbesondere für ein menschliches Auge.

In der Augenchirurgie existieren bereits spezielle Anwendungen, bei denen die Linse eines Auges durch eine Kunstlinse ersetzt wird. Bei der sogenannten Kataraktchirurgie wird eine - beispielsweise durch den grauen Star getrübte - Augenlinse durch eine Kunstlinse ersetzt. Die Linse eines Auges befindet sich in einer dünnen Umhüllung, der sogenannten Linsenkapsel. Zur Entfernung der Augenlinse wird durch einen dünnen Schnitt in die Linsenkapsel ein Zugang zur Augenlinse geschaffen und die Augenlinse mit einem mikrochirurgischen Gerät zunächst in kleine Einzelstücke zerteilt, die dann mittels einer Absaugvorrichtung entfernt werden. Anschließend wird eine Kunstlinse eingesetzt.

Die Lehre der US 5443506 bildet den Oberbegriff des Anspruchs 1.

Der vorliegenden Erfindung liegt die Aufgabe zugrunde, eine künstliche Linse für ein Auge zu schaffen, bei der sich die Brennweite verändern läßt. Diese Aufgabe wird erfindungsgemäß gelöst durch die künstliche Linse mit den Merkmalen gemäß dem unabhängigen Patentanspruch 1.

Bei der künstlichen Linse gemäß der vorliegenden Erfindung handelt es sich um ein Linsenimplantat, das anstelle der natürlichen Augenlinse in das Auge eingesetzt werden kann. Erfindungsgemäß ist vorgesehen, dass die künstliche Linse zwei oder mehr formflexible Medien aufweist. "Formflexibel" bedeutet im Lichte der vorliegenden Beschreibung, dass die Medien keine starre Oberfläche aufweisen, sondern dass sich die Medien in ihrer Form verändern können. Erfindungsgemäß ist weiterhin vorgesehen, dass die beiden formflexiblen Medien in direktem Kontakt zueinander stehen.

Die Erfindung ist nicht auf eine bestimmte Anzahl unterschiedlicher formflexibler Medien pro künstlicher Linse, auf bestimmte Materialien für die formflexiblen Medien oder auf bestimmte Zustände der formflexiblen Medien beschränkt. Diese können je nach Anwendungsfall und den gewünschten optischen Eigenschaften für die künstliche Linse gezielt ausgewählt werden. Einige nicht ausschließliche Beispiele hierzu werden im weiteren Verlauf der Beschreibung näher erläutert.

Durch die Verwendung formflexibler Medien, die die Linsenelemente der künstlichen Linse bilden, wird eine verstellbare Linse geschaffen. Insbesondere wird es nunmehr möglich, eine in ihrer Fokallänge beziehungsweise Brennweite veränderlich einstellbare Linse zu schaffen, die als Kunstlinse in das Auge eingesetzt werden kann. Dies führt zu einer Reihe neuartiger, vorteilhafter Anwendungsgebiete, wie im weiteren Verlauf der Beschreibung noch näher erläutert wird.

Vorteilhaft befinden sich die wenigstens zwei formflexiblen Medien in einem Aufnahmebehälter, der dann den Linsenkörper der künstlichen Linse bildet. Der Aufnahmebehälter kann auf unterschiedlichste Weise ausgebildet sein. Einige nicht ausschließliche Beispiele hierzu werden im weiteren Verlauf der Beschreibung näher erläutert.

Von besonderem Vorteil ist, dass die künstliche Linse durch den Einsatz der formflexiblen Medien als Linsenelemente nunmehr zur Akkommodation geeignet ausgebildet ist. "Akkommodation" bedeutet generell die Einstellung des Auges auf eine bestimmte Entfernung. Bei einem normalsichtigen Auge in Ruhestellung liegt der Fernpunkt im Unendlichen. Akkommodation ist die Fähigkeit des Auges, unter Zunahme der Brechkraft nahe gelegene Objekte auf der Netzhaut scharf abzubilden. Akkommodation ist somit die Brechkraftänderung einer Linse zum "Scharfsehen" von Objekten in wechselnden Entfernungen. Dies wird auch als Akkommodationsbreite bezeichnet. Diese nimmt mit zunehmendem Alter ab. Die Minderung der Akkommodationskraft ist die Ursache der Alterssichtigkeit.

Durch den Einsatz einer künstlichen Linse gemäß der vorliegenden Erfindung kann nunmehr die Minderung der Akkommodationskraft ausgeglichen werden, da bei dieser eine Verstellbarkeit von deren Brennweite möglich ist.

Vorzugsweise kann die künstliche Linse eine definierte voreingestellte Brechkraft aufweisen. Dadurch wird erreicht, dass die künstliche Linse in einem bestimmten Entfernungsbereich, der auf die definierte voreingestellte Brechkraft abgestimmt ist, ein Scharfsehen ermöglicht. Je nach Person, die eine solche künstliche Linse trägt, beziehungsweise nach deren Vorlieben und Anforderungen, kann die voreingestellte Brechkraft unterschiedlich sein, so dass die voreingestellten Entfernungsbereiche, in dem ein Scharfsehen möglich ist, personenbezogen unterschiedlich sein können.

In weiterer Ausgestaltung kann die künstliche Linse einen definierten Dynamikbereich der Brechkraft aufweisen. Der Dynamikbereich gibt an, um wie viel Dioptrien sich die Brechkraft der künstlichen Linse verändern kann. Die Erfindung ist nicht auf bestimmte Dynamikbereiche beschränkt. Vorteilhaft kann der Dynamikbereich mindestens 1.5 Dioptrien, bevorzugt mindestens 2.5 Dioptrien, ganz besonders bevorzugt mindestens 4 Dioptrien umfassen.

Wenn ein Fokussieren der künstlichen Linse um die definiert voreingestellte Brechkraft innerhalb des Dynamikbereichs gewünscht ist, kann dies durch eine geeignete Ansteuereinrichtung erfolgen, die im weiteren Verlauf der Beschreibung noch näher erläutert wird. Wenn ein Scharfsehen außerhalb der definierten voreingestellten Brechkraft beziehungsweise des definierten Dynamikbereichs der künstlichen Linse gewünscht ist, kann dies - insbesondere durch Ergänzung - einer geeigneten Sehhilfe, beispielsweise einer Brille, ermöglicht werden.

Die künstliche Linse weist insbesondere eine voreingestellte Brechkraft auf, die vorzugsweise einen solchen Werte aufweist, der - bei einem gegebenen Dynamikbereich der künstlichen Linse - ein Scharfsehen in einem bevorzugten und/oder möglichst großen Entfernungsbereich ermöglicht.

Nachfolgend wird zunächst ein Beispiel für einen bevorzugten Entfernungsbereich beschrieben. Beispielsweise wird ein Bildschirmarbeiter bei einem geringen Dynamikbereich der künstlichen Linse (weniger als 2 bis 3 Dioptrien) möglicherweise einen voreingestellten Wert der Brechkraft bevorzugen, der ihm ein scharfes Sehen im Bereich von einigen Zentimetern bis zu wenigen Metern ermöglicht. Möglicherweise wird er eine voreingestellte Brechkraft bevorzugen, die auf die Distanz zum Bildschirm ein scharfes Sehen ohne Aktivierung einer Ansteuereinrichtung erlaubt.

Bei größeren Dynamikbereichen (beispielsweise ab 3 bis 4 Dioptrien) kann eine Brechkraft für das Sehen auf größere Entfernungen (Unendlich) voreingestellt werden, was dem natürlichen Zustand einer natürlichen Augenlinse bei entspanntem Ciliar-Muskel entspricht und immer noch ein vernünftiges Sehen im Nahbereich erlaubt.

Bevorzugt kann die künstliche Linse so voreingestellt sein, dass sie - ohne Aktivierung einer Ansteuereinrichtung - ein Scharfsehen in Unendlich ermöglicht.

Vorteilhaft wird die Brechkraft der Hornhaut mit berücksichtigt. Deren eventuelle Fehler können durch die künstliche Linse kompensiert werden, indem die künstliche Linse eine konstante Zusatzbrechkraft zur Verfügung stellt. Diese konstante Zusatzbrechkraft braucht nicht radialsymmetrisch zu sein. So kann beispielsweise ein Astigmatismus des Auges durch eine Brechkraft geheilt/kompensiert werden, die in X-Richtung anders als in Y-Richtung ist.

Der Bereich des Scharfsehens kann je nach Anwendungsfall unterschiedlich gewählt werden, sollte aber irgendwo im Bereich zwischen 1 cm und Unendlich liegen. Natürlich kann die Brechkraft auch in solch einer Weise voreingestellt sein, dass ein Scharfsehen in einer Entfernung ermöglicht wird, die einem Unterbereich des zuvor genannten Entfemungsbereichs entspricht.

Die künstliche Linse weist vorteilhaft einen den Linsenkörper bildenden Aufnahmebehälter auf, in dem zwei oder mehr formflexible Medien vorgesehen sind. Die formflexiblen Medien sind in dem Aufnahmebehälter räumlich fixiert und berühren sich an wenigstens einer Grenzfläche. Die Fixierung kann über geeignete Fixiermittel erfolgen. Einige nicht ausschließliche Beispiele geeigneter Fixiermittel werden im weiteren Verlauf der Beschreibung näher erläutert. Dabei können beispielsweise formflexible Medien verwendet werden, die nicht mischbar sind.

Weiterhin können Mittel zum Ändern der Größe und/oder Form der Grenzfläche(n) zwischen den formflexiblen Medien vorgesehen sein. Grundsätzlich ist die Erfindung nicht auf bestimmte Medientypen beschränkt. Wichtig ist lediglich, dass die Medien formflexibel sind.

Vorteilhaft kann eine Ansteuereinrichtung zum Ansteuern der künstlichen Linse vorgesehen sein. Mit einer solchen Ansteuereinrichtung kann die künstliche Linse, beziehungsweise können die Mittel zum Ändern der Größe und/oder Form der Grenzfläche(n) in der gewünschten Weise angesteuert werden.

Die Ansteuerung der künstlichen Linse beziehungsweise der Mittel zum Ändern der Größe und/oder Form der Grenzfläche(n) kann auf unterschiedliche Weise erfolgen. Beispielsweise ist es denkbar, dass eine direkte Ansteuerung über die Augenmuskeln, etwa die Ciliar-Muskeln erfolgt. In diesem Fall stellt der Augenmuskel die Ansteuereinrichtung dar und die künstliche Linse, beziehungsweise die Mittel zum Ändern der Größe und/oder Form der Grenzfläche(n) sind mit dem Augenmuskel verbunden beziehungsweise verbindbar.

Ebenso ist es denkbar, dass die Ansteuereinrichtung zur manuellen oder automatischen Ansteuerung von außen ausgebildet ist. Beispielsweise kann die Ansteuereinrichtung ein Stellglied aufweisen und die Ansteuerung über das - insbesondere innere - Stellglied erfolgen. Dabei ist die Erfindung nicht auf bestimmte Typen von Stellgliedern beschränkt. Beispielsweise kann es sich hierbei um Mikroantriebe, Mikropumpen, Mikromotoren und dergleichen handeln. Darüber hinaus ist auch eine Ansteuerung in Kopplung mit einem Autofokussystem möglich. In diesem Fall kann die Ansteuereinrichtung ein Autofokussystem aufweisen oder als ein solches Autofokussystem ausgebildet sein. Das Autofokussystem gibt dann geeignete Stellsignale an die künstliche Linse weiter. Autofokussysteme an sich sind aus dem Stand der Technik bereits bekannt, so dass eine detaillierte Erläuterung an dieser Stelle entfallen kann.

Wie oben bereits erwähnt wurde, ist die Erfindung nicht auf bestimmte Ausgestaltungsformen des Aufnahmebehälters beschränkt. Beispielsweise kann vorgesehen sein, dass wenigstens eine Begrenzung des Aufnahmebehälters eine zumindest bereichsweise gewölbte Kontur aufweist. Dadurch kann die Kontur der zu ersetzenden Augenlinse nachempfunden werden. Bei einer Begrenzung des Aufnahmebehälters handelt es sich beispielsweise um eine Behälterwand.

Vorteilhaft kann/können eine oder mehrere Begrenzungen des Aufnahmebehälters zumindest bereichsweise transparent ausgebildet sein, um einen Lichtdurchtritt zu ermöglichen.

Beispielsweise kann wenigstens eines der formflexiblen Medien zumindest bereichsweise transparent ausgebildet sein. Vorteilhaft ist jedoch vorgesehen, dass sämtliche formflexiblen Medien zumindest teilweise transparent ausgebildet sind.

Vorteilhaft kann wenigstens ein formflexibles Medium zumindest bereichsweise an wenigstens einer Anlagefläche innerhalb des Aufnahmebehälters anliegen. Dabei kann sich die Anlagefläche an unterschiedlichen Orten innerhalb des Aufnahmebehälters befinden, so dass die Erfindung nicht auf bestimmte Anordnungs- beziehungsweise Ausbildungsvarianten beschränkt ist. Nachfolgend werden hierzu einige nicht ausschließliche Beispiele beschrieben. Bei der Anlagefläche kann es sich beispielsweise um zumindest einen Teilbereich einer Behälteraußenwand, etwa den Behälterboden und/oder ein Deckelelement und/oder wenigstens eine Seitenwand handeln. Bei der letztgenannten Variante ist insbesondere eine Ausgestaltung realisierbar, bei der das formflexible Medium nicht den Behälterboden berührt, sondern nur an den seitlichen Wänden anliegt. Natürlich sind auch Ausgestaltungsvarianten denkbar, bei denen es sich bei der genannten Anlagefläche um zumindest einen Teilbereich einer im Aufnahmebehälter-Innenraum befindlichen Zwischenschicht handelt. Dort, wo das formflexible Medium an der Anlagefläche des Aufnahmebehälters anliegt, ist dieses vorzugsweise aus einem transparenten Material gebildet, sodass von außen in den Aufnahmebehälter eintretendes Licht durch die Behälterwände und die im Aufnahmebehälter befindlichen transparenten formflexiblen Medien hindurchstrahlen kann.

Die Erfindung ist nicht auf bestimmte Medientypen beschränkt. Beispielsweise kann wenigstens ein formflexibles Medium eine Flüssigkeit sein. Beispielsweise kann es sich dabei um Wasser, um Wasser mit Salzzusätzen und dergleichen handeln. Wenigstens ein formflexibles Medium kann beispielsweise als Öl ausgebildet sein. Natürlich können die formflexiblen Medien auch in anderer Weise ausgebildet sein. Wichtig ist lediglich, dass die Medien formflexibel sind. Daher ist es beispielsweise auch denkbar, dass wenigstens ein formflexibles Medium gelartig ausgebildet ist.

Vorteilhaft kann vorgesehen sein, dass wenigstens eines der formflexiblen Medien in Form eines oder mehrerer Tropfen ausgebildet ist. Dabei ist unter einem Tropfen generell eine kleine Mediummenge von zumindest bereichsweise kugeliger oder länglich runder Form zu verstehen.

Vorzugsweise können die formflexiblen Medien die gleiche oder annähernd die gleiche Dichte aufweisen, um Gravitationseffekte auszuschließen.

In weiterer Ausgestaltung kann vorgesehen sein, dass die formflexiblen Medien in einem bestimmten Temperaturbereich die gleiche oder annähernd die gleiche Dichte aufweisen. Dieser Temperaturbereich ist insbesondere auf die Körpertemperatur der Person, die eine derartige künstliche Linse trägt, abgestimmt. Beispielsweise kann der Temperaturbereich zwischen 30°C und 45°C, vorzugsweise zwischen 35°C und 40°C, ganz besonders bevorzugt zwischen 36°C und 38°C liegen.

Weiterhin können wenigstens zwei der formflexiblen Medien durchaus unterschiedliche optische Eigenschaften, beispielsweise unterschiedliche optische Indizes, aufweisen. Dabei kann beispielsweise vorgesehen sein, dass diese unterschiedliche Brechungszahlen aufweisen. So ist gemäß einem vorteilhaften - nicht ausschließlichen - Beispiel vorgesehen, dass ein formflexibles Medium eine niedrige Brechzahl (Brechungsindex) aufweist, während ein anderes formflexibles Medium eine hohe Brechzahl aufweist.

Wenn die formflexiblen Medien an einer transparenten Anlagefläche anliegen, beispielsweise an einer zumindest teilweise transparenten Begrenzung des Aufnahmebehälters, weisen diese transparenten Bereiche der Anlagefläche beziehungsweise der Behälterbegrenzung vorzugsweise die gleiche oder eine ähnliche Brechzahl wie das anliegende formflexible Medium auf. Dadurch werden gebrochene Strahlengänge und unerwünschte Reflexionen vermieden. Natürlich können auch Ausgestaltungen realisiert werden, bei denen die jeweiligen Brechzahlen unterschiedlich sind. Dies kann beispielsweise dazu genutzt werden, um besondere optische Effekte einzustellen.

Vorteilhaft kann vorgesehen sein, dass ein formflexibles Medium über die Mittel zum Ändern der Grenzfläche(n) derart in Richtung des jeweils anderen formflexiblen Mediums verschoben wird, dass sich die Krümmung wenigstens einer Grenzfläche zwischen den beiden formflexiblen Medien ändert.

Die Erfindung ist nicht auf bestimmte Ausgestaltungsformen für die Mittel zur Änderung der Grenzfläche(n) zwischen den formflexiblen Medien beschränkt. Nachfolgend werden hierzu einige nicht ausschließliche Beispiele angegeben.

Beispielsweise können die Mittel zum Ändern der Grenzfläche(n) ringförmig um eine lichte Öffnung herum angeordnet sein. Dabei können die ringförmigen Mittel je nach Ausführungsform einteilig oder aber auch mehrteilig ausgebildet sein.

Die lichte Öffnung kann vorzugsweise zumindest dem maximalen Pupillendurchmesser des Auges entsprechen, für das die künstliche Linse bestimmt ist.

Beispielsweise kann vorgesehen sein, dass die Mittel zum Ändern der Größe und/oder Form der Grenzfläche(n) zwischen den formflexiblen Medien auf der Basis des Elektrobenetzens (Electrowetting) ausgebildet sind.

Das Prinzip des Elektrobenetzens über die Erzeugung eines elektrischen Feldes kann nun vorsehen, dass ein erstes formflexibles Medium und ein zweites formflexibles Medium eine unterschiedliche elektrische Leitfähigkeit aufweisen. Das Medium mit der geringeren elektrischen Leitfähigkeit, beispielsweise ein Öl, kann zwischen dem Medium mit der größeren elektrischen Leitfähigkeit, beispielsweise Wasser oder Wasser mit Zusätzen sowie wenigstens einer Elektrode angeordnet sein. Dabei kann beispielsweise vorgesehen sein, dass das Medium mit der geringeren elektrischen Leitfähigkeit auf einer Oberfläche eines Substrats angeordnet ist, während auf der anderen Oberfläche des Substrats die wenigstens eine Elektrode angeordnet ist. Wenn nun ein elektrisches Feld zwischen der wenigstens einen Elektrode und dem Medium mit der größeren elektrischen Leitfähigkeit angelegt wird, wird dadurch die Grenzfläche zwischen den beiden formflexiblen Medien verändert.

Dabei wirken die Mittel zum Ändern der Grenzfläche auf ein elektrisch leitendes Medium ein. Durch Anlegen einer elektrischen Spannung wird die Benetzbarkeit einer Oberfläche, an der das elektrisch leitende Medium anliegt, durch dieses elektrisch leitende Medium verändert, sodass sich dadurch indirekt auch die Kontur des elektrisch isolierenden Mediums verändern kann.

Eine Umsetzung dieses Phänomens ist beispielsweise aus der US-A-5,659,330 bekannt. Darin wird eine Anzeigeeinrichtung beschrieben, bei der einzelne Tropfen einer Leiterflüssigkeit auf einer Isolationsschicht angeordnet sind. Unterhalb dieser Isolationsschicht sind Elektroden vorhanden. Durch die selektive Erzeugung eines elektrischen Feldes kann die Form eines jeden Tropfens der Leiterflüssigkeit variiert werden, wodurch ein farbiges Pixel eines Bildes erzeugt wird.

Eine Lösung, wie die das Prinzip des Elektrobenetzens generell auch im Bereich von Linsenelementen eingesetzt werden kann, ist in der DE 698 04 119 T2 beschrieben. Die darin geschilderte Erfindung liegt auf dem Gebiet verstellbarer Fokuslinsen, und dort insbesondere auf dem Gebiet flüssiger Linsen mit einem verstellbaren, elektrisch gesteuerten Fokus. Dabei soll es mit dem dort beschriebenen Linsenelement möglich sein, den Fokus mit Hilfe des sogenannten "Elektrobenetzens" kontinuierlich zu verstellen. Gemäß dieser bekannten Lösung wird ein optisches Element zum veränderbaren Einstellen der Fokallänge bereitgestellt. Bei der Fokallänge handelt es sich generell um den Abstand des Brennpunkts zur Ebene des optischen Elements, beispielsweise zur Linsenebene.

Das bekannte optische Element besteht aus einem Aufnahmebehälter, der eine erste, elektrisch leitfähige Flüssigkeit sowie einen Tropfen einer zweiten, isolierenden Flüssigkeit beinhaltet. Die beiden Flüssigkeiten sind nicht mischbar und im Aufnahmebehälter räumlich fixiert. Die beiden Flüssigkeiten berühren einander an einer Grenzfläche. Der Tropfen der zweiten Flüssigkeit ist konzentrisch um die optische Achse des optischen Elements herum angeordnet, wobei die optische Achse ebenfalls durch einen transparenten Bereich des Bodens des Aufnahmebehälters verläuft. Die erste, elektrisch leitfähige Flüssigkeit liegt zumindest bereichsweise an Elektroden an, die sich innerhalb des Aufnahmebehälters befinden. Darüber hinaus ist wenigstens eine weitere Elektrode vorgesehen, die außerhalb des Behälterbodens angeordnet ist. Dabei ist diese weitere Elektrode auf der dem Behälterinnenraum abgewandten Oberfläche des Behälterbodens angeordnet.

Schließlich weist das bekannte optische Element Mittel zum Ändern der Grenzfläche zwischen den Flüssigkeiten auf. Über diese Mittel kann ein elektrisches Feld zwischen den Elektroden erzeugt werden. Dadurch ändert sich die Benetzbarkeit der mit der ersten, leitfähigen Flüssigkeit überdeckten Fläche, so dass sich darüber auch die Form des Tropfens der zweiten, isolierenden Flüssigkeit verändert. Durch die Variation der Größe und/oder Form der Grenzfläche zwischen den beiden Flüssigkeiten kann der Fokus des optischen Elements kontinuierlich verstellt werden.

Ähnliche Lösungen sind beispielsweise auch aus der WO 03/071335 A2 sowie der WO 03/069380 A1 bekannt.

In anderer Ausgestaltung können die Mittel zum Ändern der Grenzfläche(n) zur Einwirkung auf wenigstens eines der formflexiblen Medien ausgebildet sein, wobei die Mittel zum Ändern der Grenzfläche(n) zum Erzeugen eines Drucks auf wenigstens eines der formflexiblen Medien ausgebildet sind und wobei ein formflexibles Medium über diese Mittel an wenigstens einer Grenzfläche in zumindest einer Vorzugsrichtung in Richtung eines jeweils anderen formflexiblen Mediums verschoben, insbesondere gedrückt, wird oder werden kann. Derartige Mittel können konstruktiv einfach und in energiesparender Weise ausgestaltet werden, wobei solche Mittel häufig nur sehr kleine Steuerspannungen benötigen. Hierbei spielt die elektrische Leitfähigkeit der Medien keine Rolle.

Durch diese Mittel wird erreicht, dass ein Druck auf das entsprechende formflexible Medium ausgeübt wird, sodass dieses an der Grenzfläche in zumindest einer Vorzugsrichtung in Richtung des jeweils anderen formflexiblen Mediums verschoben - insbesondere gedrückt - werden kann. Bei dieser Vorzugsrichtung kann es sich in vorzugsweise um die optische Achse der künstlichen Linse handeln. Die Änderung der Brennweite der künstlichen Linse und damit die Änderung der Fokallänge erfolgt dann quasi durch das Herausdrücken des einen formflexiblen Mediums in Richtung des anderen formflexiblen Mediums.

Dabei kann beispielsweise vorgesehen sein, dass die Mittel zum Ändern der Grenzfläche(n) zur Einwirkung auf ein zweites formflexibles Medium ausgebildet sind und dass das zweite formflexible Medium über diese Mittel an wenigstens einer Grenzfläche in zumindest einer Vorzugsrichtung in Richtung eines ersten formflexiblen Mediums verschoben, insbesondere gedrückt, wird oder werden kann. Zusätzlich oder alternativ ist auch denkbar, dass die Mittel zum Ändern der Grenzfläche(n) zur Einwirkung auf ein erstes formflexibles Medium ausgebildet sind und dass das erste formflexible Medium über diese Mittel an wenigstens einer Grenzfläche in zumindest einer Vorzugsrichtung in Richtung eines zweiten formflexiblen Mediums verschoben, insbesondere gedrückt, wird oder werden kann.

In weiterer Ausgestaltung kann auch vorgesehen sein, dass sich zwei formflexible Medien an zwei Grenzflächen berühren und dass ein formflexibles Medium über die Mittel zum Ändern der Grenzflächen an einer oder beiden Grenzflächen in zumindest einer Vorzugsrichtung in Richtung des jeweils anderen formflexiblen Mediums verschoben, insbesondere gedrückt, wird oder werden kann. In einem solchen Fall ist es beispielsweise auch möglich, dass zwei Vorzugsrichtungen - für jede Grenzfläche eine - gewählt werden können. Hierbei können die Vorzugsrichtungen gemäß einer vorteilhaften Weiterbildung entgegengesetzt ausgerichtet sein.

Dabei kann vorteilhaft vorgesehen sein, dass ein formflexibles Medium über die Mittel zum Ändern der Grenzfläche(n) derart in Richtung eines anderen formflexiblen Mediums verschoben, insbesondere gedrückt, wird, dass sich die Krümmung wenigstens einer Grenzfläche zwischen den beiden formflexiblen Medien ändert.

Gemäß einer weiteren Ausgestaltungsform kann wenigstens ein formflexibles Medium allseitig von einem anderen formflexiblen Medium umschlossen sein, wobei die Mittel zum Ändern der Grenzfläche zur Einwirkung auf wenigstens ein formflexibles Medium ausgebildet sind und wobei die Mittel zum Ändern der Grenzfläche(n) zum Erzeugen eines Drucks auf wenigstens eines der formflexiblen Medien ausgebildet sind.

Bei dieser Lösung ist eine besondere Anlagefläche nicht erforderlich. Vielmehr ist ein allseitig umschlossenes erstes formflexibles Medium, das im Ausgangszustand vorteilhaft eine kugelartige Konfiguration aufweist, von allen Seiten von einem anderen, zweiten formflexiblen Medium umgeben. Bei dem ersten formflexiblen Medium kann es sich beispielsweise wiederum um Wasser und bei dem zweiten formflexiblen Medium vorteilhaft um ein geeignetes Öl handeln. Die beiden formflexiblen Medien weisen vorteilhaft die gleiche Dichte ab, damit das zweite formflexible Medium innerhalb des ersten formflexiblen Mediums in Position gehalten wird und nicht absinken kann, das heißt, damit Gravitationseffekte wirksam ausgeschlossen werden können.

Die Mittel zum Ändern der Grenzfläche üben nun einen Druck auf wenigstens eines der formflexiblen Medien aus. Wenn der Druck auf ein erstes formflexibles Medium ausgeübt wird, wird der Druck vom ersten auf ein zweites formflexibles Medium übertragen, so dass dieses an der Druck-Einwirkstelle komprimiert wird, was die Größe und/oder Form der Grenzfläche zwischen den beiden formflexiblen Medien verändert. Beispielsweise kann das zweite formflexible Medium von der ursprünglich kugelartigen Ausgangsform in eine elliptische Konfiguration gebracht werden. Wenn der Druck auf das zweite formflexible Medium ausgeübt wird, wird sich dieses gegen das erste formflexible Medium ausdehnen, so dass sich auch in diesem Fall die Größe und/oder Form der Grenzfläche zwischen den beiden formflexiblen Medien ändern wird.

Beispielsweise können die Mittel zum Ändern der Grenzfläche(n) als mechanische Mittel ausgebildet sein, beispielsweise in Form einer Kolbeneinrichtung, einer Stempeleinrichtung, einer Zylindereinrichtung, und dergleichen.

In anderer Ausgestaltung können die Mittel zum Ändern der Grenzfläche(n) beispielsweise auch in Form wenigstens einer ansteuerbaren Membran ausgebildet sein.

In den beiden vorgenannten Fällen bietet sich beispielsweise eine direkte Ansteuerung über die Augenmuskeln, etwa die Ciliar-Muskeln an. Sofern die Mittel zum Ändern der Grenzfläche(n) auf magnetischer, elektrischer oder elektromagnetischer Basis arbeiten, kann beispielsweise eine manuelle Ansteuerung von außen erfolgen, etwa indem ein Magnet verschoben wird, indem ein Elektromagnet in geeigneter Weise angesteuert wird, oder dergleichen.

Dabei ist die Erfindung selbstverständlich ebenfalls nicht auf bestimmte Antriebsarten für die Mittel zum Ändern der Grenzfläche beschränkt. So ist es etwa möglich, dass die Mittel elektrisch betätigbar ausgebildet sind. Die in einem solchen Fall benötigten Spannungen liegen im unteren Voltbereich. Daher sind solche Mittel medizinisch besonders unbedenklich, energiesparend und kosteneffizient einsetzbar. Selbstverständlich sind auch andere Antriebsarten für die Mittel zum Ändern der Grenzfläche möglich. So ist es beispielsweise denkbar, dass diese magnetisch und/oder elektromagnetisch und/oder pneumatisch und/oder hydraulisch und/oder piezoelektrisch, oder dergleichen betätigbar ausgebildet sind.

In einer vorteilhaften Ausführungsform kann die Betätigung der Mittel zum Ändern der Grenzfläche beispielsweise über den Sehnerv des Auges gesteuert werden. In einem solchen Fall könnte die Betätigung der Mittel, insbesondere bei einer mechanischen Betätigung, auch über Muskelpartien rund um das Auge erfolgen.

Die räumliche Fixierung der formflexiblen Medien innerhalb des Aufnahmebehälters kann mittels geeigneter Fixiermittel erfolgen. Dies ist insbesondere deshalb von Bedeutung, da ein Strahlengang definiert durch die künstliche Linse hindurchlaufen soll. Die räumliche Fixierung erfolgt vorzugsweise mittels dazu geeigneter Fixiermittel. Dabei ist die Erfindung jedoch nicht auf bestimmte Typen von Fixiermitteln beschränkt.

Vorteilhaft können die Fixiermittel in Form einer oder mehrerer unterschiedlicher Oberflächenbeschichtung(en) innerhalb des Aufnahmebehälters ausgebildet sein. Alternativ oder zusätzlich können die Fixiermittel in Form geometrischer Ausgestaltungen zumindest von Bereichen des Aufnahmebehälters ausgebildet sein. Hierbei kann es sich beispielsweise um Vorsprünge, Hinterschneidungen, Ausnehmungen und dergleichen handeln.

Die Fixiermittel können beispielsweise an wenigstens einer Wandung des Aufnahmebehälters beziehungsweise an wenigstens einem Bereich wenigstens einer Wandung des Aufnahmebehälters angeordnet und/oder ausgebildet sein. Ebenso ist es auch möglich, dass die Fixiermittel innerhalb des Aufnahmebehälters, etwa im Bereich einer als Zwischenschicht dienenden Anlagefläche oder dergleichen angeordnet oder ausgebildet sind.

Nachfolgend werden einige vorteilhafte, nicht ausschließliche Ausführungsbeispiele für geeignete Fixiermittel beschrieben.

Beispielsweisekönnen die Fixiermittel in Form einer besonderen Oberflächengestaltung der Anlagefläche und/oder in Form einer besonderen Oberflächenbeschaffenheit und/oder in Form einer besonderen Oberflächenbeschichtung der Anlagefläche und/oder des Aufnahmebehälters ausgebildet sein. Hierbei kann es sich beispielsweise um hydrophile und/oder hydrophobe Beschichtungen handeln. Die Oberfläche ist dabei vorteilhaft so gestaltet, dass sie das anliegende formflexible Medium in Position halten kann. Die besondere Oberflächenbeschaffenheit kann beispielsweise mittels einer besonderen Oberflächenbeschichtung realisiert werden. Vorteilhaft kann eine besondere Oberflächenbeschaffenheit bezüglich der Benetzbarkeit vorgesehen sein. Dabei ist die Erfindung natürlich nicht auf die genannten Beispiele beschränkt.

So kann etwa vorgesehen sein, dass ein Teil der Beschichtung aus einem hydrophoben und ein Teil der Beschichtung aus einem hydrophilen Material bestehen, und dass die beiden unterschiedlichen Materialien entlang einer Grenzlinie aneinandergrenzen. Diese Grenzlinie, die statisch, dass heißt unveränderlich ist, bildet auch eine Grenzlinie zwischen den beiden formflexiblen Medien, von denen eines dann vorteilhaft Wasser oder eine wässrige Lösung, und eines zum Beispiel Öl sein kann. Durch die entsprechende Beschichtung einer Fläche kann dann erreicht werden, dass sich die Position der formflexiblen Medien an der Fläche nicht verändert, die Medien in diesem Bereich somit fixiert sind. Eine Veränderung der Grenzfläche zwischen den beiden Medien ist dann in der wie weiter oben beschriebenen Weise nur im Bereich der freien Grenzfläche zwischen den beiden Medien möglich, an denen sich die beiden Medien direkt berühren und die sich in einem Abstand zu der beschichteten Fläche befindet.

Beispielsweise ist denkbar, dass die Fixierung der formflexiblen Medien durch eine geeignete Wahl der Oberflächenmaterialien und/oder lokale Oberflächenbeschichtungen innerhalb des Aufnahmebehälters, beispielsweise der Begrenzung beziehungsweise Wand des Aufnahmebehälters, erfolgt. Ebenso kann die räumliche Fixierung der formflexiblen Medien über das Anlegen einer geeigneten, vorzugsweise festgelegten Spannung, erfolgen. In diesem Fall ist es vorteilhaft, wenn ein erstes formflexibles Medium als elektrisch leitfähiges Medium und ein zweites formflexibles Medium als elektrisch isolierendes Medium ausgebildet ist. Natürlich ist es auch denkbar, die räumliche Fixierung über die konstruktive Ausgestaltung der Begrenzungen und/oder Zwischenschicht(en) innerhalb des Aufnahmebehälters zu erreichen, beispielsweise in dem diese mit geeigneten Vorsprüngen, Kanten, Hinterschneidungen, Aussparungen und dergleichen versehen sind.

Die Fixiermittel haben generell die Aufgabe, dass die Position der formflexiblen Medien innerhalb des Aufnahmebehälters unverändert bleibt, sodass über die künstliche Linse insbesondere ein definierter Strahlengang erzeugt werden kann. Die Position ist dabei bezüglich der Wandung des Aufnahmebehälters und/oder bezüglich einer Anlagefläche innerhalb des Aufnahmebehälters zu verstehen.

Vorteilhaft kann vorgesehen sein, dass in der Anlagefläche des Aufnahmebehältersbeispielsweise in einer Zwischenschicht - an der ein formflexibles Medium anliegt, eine Öffnung vorgesehen ist, und dass das formflexible Medium im Bereich dieser Öffnung fixiert ist. Die Öffnung, die vorteilhaft zumindest dem maximalen Pupillendurchmesser des Auges entspricht, für das die künstliche Linse bestimmt ist, kann sich insbesondere um eine optische Achse der künstlichen Linse herum erstrecken, sodass ein Lichtstrahl durch diese Öffnung und anschließend durch die formflexiblen Medien hindurchtreten kann. Insbesondere sind im Bereich der Öffnung dazu alle in Frage kommenden Komponenten der künstlichen Linse transparent ausgebildet.

In weiterer Ausgestaltung können auch Mittel zum Stabilisieren (Stabilisierungsmittel) der Oberflächenstruktur wenigstens eines der formflexiblen Medien vorgesehen sein. Diese Stabilisierungsmittel können einem unerwünschten Zerfall der Konturen der formflexiblen Medien entgegenwirken.

Die Stabilisierungsmittel können die Oberflächenstrukturen der formflexiblen Medien zusammenhalten, ohne dass dabei die optische Qualität der künstlichen Linse wesentlich beeinträchtigt wird. Dabei erfolgt die Stabilisierung vorzugsweise durch den Einsatz äußerer und/oder innerer Strukturen. Die Stabilisierungsmittel können auf unterschiedlichste Art und Weise ausgebildet sein, so dass die Erfindung nicht auf bestimmte Ausgestaltungsformen beschränkt ist. Nachfolgend werden hierzu einige nicht ausschließliche Beispiele beschrieben.

Vorteilhaft können die Stabilisierungsmittel zumindest teilweise im Bereich der Grenzfläche(n) zwischen zwei - benachbarten - formflexiblen Medien vorgesehen sein. Dabei soll jedoch auch mit umfasst sein, dass zumindest zeitweilig ein gewisser Abstand zwischen den Stabilisierungsmitteln und der/den Grenzfläche(n) besteht. Dieser Abstand ist dabei so zu wählen, dass eine Zerstörung der Oberflächenstruktur der formflexiblen Medien nicht auftreten kann.

Beispielsweise können die Stabilisierungsmittel als wenigstens eine - insbesondere elastische - Folie, insbesondere als Lochfolie, oder dergleichen ausgebildet sein. In diesem Fall handelt es sich bei den Stabilisierungsmitteln um eine Stabilisierungsfolie. Wenn die Stabilisierungsmittel als durchgängige Folie ausgebildet sind, ist es nicht erforderlich, dass die formflexiblen Medien nicht mischbar sind. In weiterer Ausgestaltung können die Stabilisierungsmittel wenigstens ein Element mit einer Maschenstruktur, insbesondere mit wenigstens einer - möglicherweise elastische - Struktur aus Fäden und/oder Bändern, oder dergleichen aufweisen. Es können auch zwei oder mehr solcher Elemente vorgesehen sein. Dies gilt übrigens auch für die als Folie ausgebildeten Stabilisierungsmittel. Bei der elastischen Ausgestaltung des wenigstens einen Elements handelt es sich um ein optionales Merkmal, das für die Durchführbarkeit der Erfindung nicht zwingend erforderlich ist. In diesem Fall handelt es sich bei den Stabilisierungsmitteln beispielsweise um ein Stabilisierungsnetz. Das Stabilisierungsnetz kann vorteilhaft als feinmaschiges Netz aus Fäden oder Bändchen ausgebildet sein, die die Oberflächenstruktur des formflexiblen Mediums zusammenhalten und die optische Qualität nicht wesentlich beeinträchtigen. Die einzelnen Fäden sind vorteilhaft nicht zu dick ausgebildet. Allerdings ist auch eine Dickenvariation über die gesamte Kontur möglich. Insbesondere dort, wo große Belastungen auf die Stabilisierungsmittel einwirken, wenn sich die Grenzfläche zwischen den formflexiblen Medien ändert, können die Fäden dicker sein als beispielsweise dort, wo eine geringere Belastung auftritt.

Die Erfindung ist nicht auf bestimmte Materialien beschränkt, aus denen die Stabilisierungsmittel hergestellt sein können. Beispielsweise handelt es sich bei geeigneten Materialien um Glasfasern, um Nylon und dergleichen.

Vorteilhaft können die Stabilisierungsmittel zumindest bereichsweise oder teilweise transparent ausgebildet sein. Dadurch wird die optische Qualität der künstlichen Linse durch die zusätzlichen Stabilisierungsmittel nicht beeinträchtigt. Grundsätzlich sind jedoch auch nicht transparente Stabilisierungsmittel denkbar, solange diese die Funktion des optischen Elements nicht beeinträchtigen, etwa, wenn diese nicht zu dick sind.

Nachfolgend wird der Einsatz von Stabilisierungsmitteln beispielhaft für eine künstliche Linse beschrieben, die als Linsenelemente zwei formflexible Medien aufweist. Selbstverständlich lässt sich die beispielhafte Beschreibung in analoger Weise auch auf künstliche Linsen übertragen, die mehr als zwei formflexible Medien aufweisen.

Die Stabilisierungsmittel können je nach Ausgestaltungsform im ersten und/oder zweiten formflexiblen Medium angeordnet sein.

Beispielsweise können die Stabilisierungsmittel innerhalb des zweiten formflexiblen Mediums angeordnet sein. In einem solchen Fall ist vorteilhaft vorgesehen, dass die Stabilisierungsmittel in einer Weise ausgebildet sind, um das zweite formflexible Medium an der/den Oberfläche(n) der Stabilisierungsmittel festzuhalten, insbesondere durch eine besondere Oberflächenbeschichtung der Stabilisierungsmittel. Beispielsweise können die Stabilisierungsmittel, etwa ein Stabilisierungsnetz, mit einem Material oberflächenbeschichtet sein, oder aus einem Material bestehen, das das zweite formflexible Medium, etwa in Form eines Öls, an der Oberfläche "festhält", wenn das Stabilisierungsnetz innerhalb des zweiten formflexiblen Mediums plaziert ist. Die Stabilisierungsmittel sind somit in einer Weise ausgestaltet, dass deren Oberfläche(n) für das zweite formflexible Medium benetzbar gemacht/gehalten wird.

Ebenso ist möglich, dass die Stabilisierungsmittel innerhalb des ersten formflexiblen Mediums angeordnet sind. In einem solchen Fall können die Stabilisierungsmittel beispielsweise in einer Weise ausgebildet sein, um das zweite formflexible Medium von der/den Oberfläche(n) der Stabilisierungsmittel zu verdrängen, insbesondere durch eine besondere Oberflächenbeschichtung der Stabilisierungsmittel. Beispielsweise können die Stabilisierungsmittel, etwa ein Stabilisierungsnetz, mit einem Material oberflächenbeschichtet sein, oder aus einem Material bestehen, das das zweite formflexible Medium, etwa in Form eine Öls, von der Oberfläche verdrängt, wenn das Stabilisierungsnetz außerhalb des zweiten formflexiblen Mediums plaziert ist. Gleichzeitig soll das andere formflexible Medium, nämlich das erste Medium, in dem sich die Stabilisierungsmittel dann befinden - etwa Wasser - nicht von den Stabilisierungsmitteln verdrängt werden.

Ebenso sind Ausführungsformen realisierbar, bei denen Stabilisierungsmittel sowohl innerhalb des ersten formflexiblen Mediums als auch innerhalb des zweiten formflexiblen Mediums angeordnet sind.

Vorzugsweise können die Stabilisierungsmittel zumindest bereichsweise eine Vorspannung aufweisen. Das bedeutet, dass die Stabilisierungsmittel mit einer vorgeformten Kontur ausgebildet sein können. Beispielsweise kann vorgesehen sein, dass diese vorgeformte Kontur an die Kontur der Grenzfläche zwischen den beiden formflexiblen Medien angepasst ist, beispielsweise im Bereich der maximalen und/oder minimalen Ausdehnung des zweiten formflexiblen Mediums.

Vorteilhaft können die Stabilisierungsmittel den gleichen oder zumindest einen ähnlichen Brechungsindex aufweisen wie das formflexible Medium, in dem sie sich befinden. Wenn sich die Stabilisierungsmittel innerhalb des zweiten formflexiblen Mediums befinden, sollte deren Material den Brechungsindex des zweiten formflexiblen Mediums, etwa eines Öls oder dergleichen, haben. Wenn sich die Stabilisierungsmittel außerhalb des zweiten formflexiblen Mediums befinden, sollten sie vorzugsweise den Brechungsindex des ersten formflexiblen Mediums, beispielsweise Wasser, haben.

Die Erfindung wird nun anhand von Ausführungsbeispielen unter Bezugnahme auf die beiliegenden Zeichnungen näher erläutert. Es zeigen:
- Figur 1: eine schematische Darstellung eines menschlichen Auges;
- Figur 2: ein erstes Ausführungsbeispiel einer künstlichen Linse gemäß der vorliegenden Erfindung;
- Figur 3: ein zweites Ausführungsbeispiel einer künstlichen Linse gemäß der vorliegenden Erfindung;
- Figur 4: eine schematische Draufsicht auf die in Figur 3 dargestellte künstliche Linse; und
- Figur 5: ein drittes Ausführungsbeispiel einer künstlichen Linse gemäß der vorliegenden Erfindung.

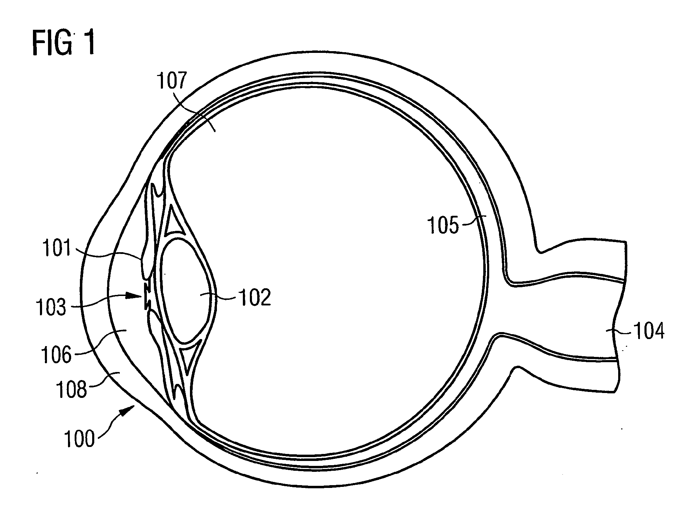

In Figur 1 ist zunächst in allgemeiner Form ein menschliches Auge 100 dargestellt. Hinter der Cornea 108 befindet sich die Iris 101 mit der Pupille 103. Die Pupille 103 liegt vor der Linse 102. Zwischen Cornea 103 und Iris 101 befindet sich die Augenkammer. Im hinteren Teil des Glaskörperraums 107 liegt die Retina 105. Weiterhin gehen von dort die Zentralgefäße 104 ab.

Mit zunehmendem Alter des Menschen nimmt die Akkommodationsbreite von dessen Auge ab. Die Minderung der Akkommodationskraft ist die Ursache der Alterssichtigkeit. "Akkommodation" bedeutet generell die Einstellung des Auges auf eine bestimmte Entfernung. Bei einem normalsichtigen Auge in Ruhestellung liegt der Fempunkt im Unendlichen. Akkommodation ist die Fähigkeit des Auges, unter Zunahme der Brechkraft nahe gelegene Objekte auf der Netzhaut scharf abzubilden.

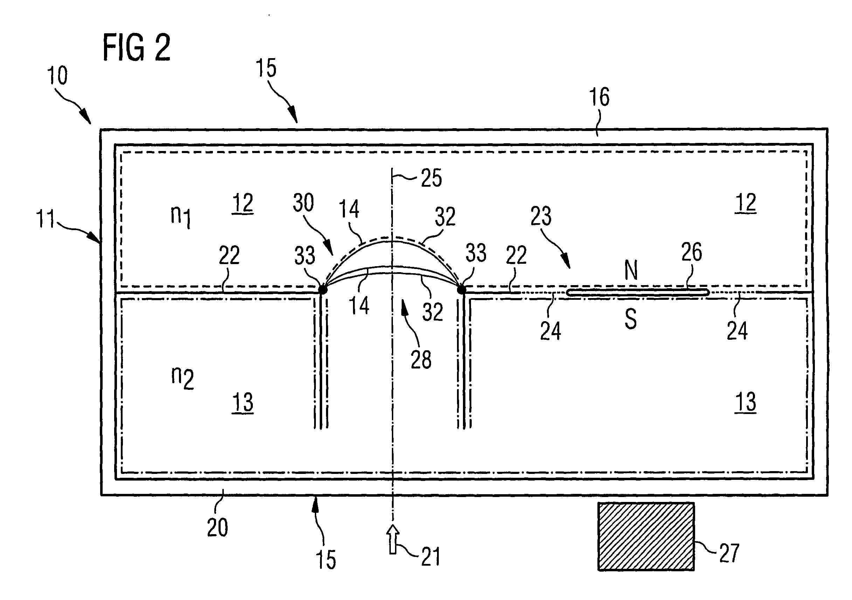

Durch den Austausch der Linse 102 durch eine künstliche Linse (Implantatlinse) gemäß der vorliegenden Erfindung, wie sie anhand einiger Ausführungsbeispiele nachfolgend im Zusammenhang mit Figuren 2 bis 5 beschrieben wird, kann nunmehr die Minderung der Akkommodationskraft ausgeglichen werden, da bei der künstlichen Linse eine Verstellbarkeit von deren Brennweite möglich ist.

In Figur 2 ist ein erstes Ausführungsbeispiel für eine künstliche Linse 10 gemäß der vorliegenden Erfindung dargestellt, bei der es sich um ein Linse mit veränderlicher Brennweite handeln soll. Bei der in Figur 2 gezeigten Darstellung handelt es sich um eine Verdeutlichung des grundlegenden Funktionsprinzips der künstlichen Linse 10 mit variabler Brennweite. Es ist selbstverständlich, dass eine derartige künstliche Linse 10 bei Verwendung als Linse 102 in einem Auge 100 (Figur 1) von der Ausgestaltung her an die Konturen des Auges anzupassen ist.

Die in Figur 2 dargestellte künstliche Linse 10 besteht zunächst aus einem Aufnahmebehälter 11, der unter anderem durch einen Behälterboden 20 und einen diesem gegenüberliegenden Behälterdeckel 16 begrenzt ist. Bei dem Behälterboden 20 und dem Behälterdeckel 16 handelt es sich um Begrenzungen 15 des Aufnahmebehälters 11. Der Aufnahmebehälter 11 stellt den Linsenkörper der künstlichen Linse 10 dar.

Senkrecht zum Behälterboden 20 verläuft die optische Achse 25, entlang derer der Strahlengang 21 eines Lichtstrahls durch die künstliche Linse 10 hindurch verläuft.

Zumindest in einem Bereich um die optische Achse 25 herum weist der Behälterboden 20 und ebenso der Behälterdeckel 16 einen transparenten Bereich auf. Natürlich ist es auch denkbar, dass der gesamte Behälterboden 20 und der gesamte Behälterdeckel 16 aus einem transparenten Material gebildet sind.

Innerhalb des Aufnahmebehälters 11 befinden sich zwei unterschiedliche, jeweils formflexible Medien 12, 13, bei denen es sich um Linsenelemente der künstlichen Linse 10 handelt. Beide Medien 12, 13 sind nicht mit einander mischbar, weisen unterschiedliche optische Eigenschaften (unterschiedliche Brechzahlen n1 und n2) auf und verfügen zumindest über eine ähnliche Dichte. Darüber hinaus sind beide Medien 12, 13 transparent. Im vorliegenden Ausführungsbeispiel kann es sich bei den formflexiblen Medien 12, 13 um Flüssigkeiten handeln, wobei beispielsweise das erste Medium 12 als Wasser und das zweite Medium 13 als Öl ausgebildet sein kann.

Innerhalb des Aufnahmebehälters 11 befindet sich in diesem eine als Anlagefläche 22 ausgebildete Zwischenschicht, die wiederum über eine Öffnung 28 verfügt. Ebenso wie der transparente Bereich des Behälterbodens 20 ist auch die Öffnung 28 innerhalb der Zwischenschicht 22 konzentrisch um die optische Achse 25 herum ausgebildet. Die Öffnung 28 kann beispielsweise dem maximalen Pupillendurchmesser des Auges entsprechen, für das die künstliche Linse 10 bestimmt ist.

Bei dem in Figur 2 dargestellten Beispiel sind solche Oberflächen innerhalb des Aufnahmebehälters 11, die mit dem ersten Medium 12 benetzt werden, durch eine gestrichelte Linie gekennzeichnet, während diejenigen Oberflächen, die mit dem zweiten Medium 13 benetzt werden, durch eine strichpunktierte Linie gekennzeichnet sind.

Die beiden Flüssigkeiten 12, 13 sind über geeignete Mittel im Aufnahmebehälter 11 räumlich fixiert, wobei die zweite Flüssigkeit 13 zumindest bereichsweise an der als Anlagefläche ausgebildeten Zwischenschicht 22 innerhalb des Aufnahmebehälters 11 anliegt. Dabei ist die zweite Flüssigkeit 13 ebenfalls im Bereich der Öffnung 28 fixiert, sodass sich die Flüssigkeit 13, die zumindest im Bereich der Grenzfläche 14 zwischen der ersten und zweiten Flüssigkeit 12, 13 eine Tropfenform aufweist, konzentrisch um die optische Achse 25 herum erstreckt.

Ein in die künstliche Linse 10 eintretender Lichtstrahl 21 durchläuft somit zunächst den transparenten Bereich des Behälterbodens 20, anschließend die zweite Flüssigkeit 13 sowie die Öffnung 28 in der Anlagefläche 22 innerhalb des Aufnahmebehälters 11, dann die erste Flüssigkeit 12 und anschließend einen transparenten Bereich des Behälterdeckels 16. Wenn der Lichtstrahl 21 über den Behälterdeckel 16 eintritt, verläuft der Strahlengang genau umgekehrt.

Eine Veränderung der Brennweite der künstlichen Linse 10 erfolgt nun in einer Weise, das die Größe und/oder Form der Grenzfläche 14 - zum Beispiel deren Krümmung - zwischen den beiden Flüssigkeiten 12, 13 verändert wird. Dies erfolgt über entsprechend ausgestaltete Mittel 23. Im vorliegenden Ausführungsbeispiel sind die Mittel 23 zum Ändern der Grenzfläche 14 in Form einer Membran 24 ausgebildet, die einen Teil der Zwischenschicht 22 bildet. An oder in der Membran 24 befindet sich wenigstens ein Magnet- oder Metallplättchen 26. Auf dieses Plättchen 26 kann mittels eines Elektromagneten 27 eingewirkt werden.

Wenn das Plättchen 26 als Magnetplättchen ausgebildet ist, kann dieses, je nach Polung, bei Betätigung des Elektromagneten 27 in Richtung des Behälterbodens 20 angezogen, oder aber in Richtung des Behälterdeckels 16 abgestoßen werden. Wenn das Plättchen 26 als Metallplättchen ausgebildet ist, wird dieses bei Betätigung des Elektromagneten 27 angezogen.

Durch die Mittel 23 zum Ändern der Grenzfläche 14 ist es nunmehr möglich, auf die Flüssigkeit 13 direkt einzuwirken. Dies geschieht in einer Weise, dass die zweite Flüssigkeit 13 an der Grenzfläche 14 zur ersten Flüssigkeit 12 in zumindest einer Vorzugsrichtung - im vorliegenden Beispiel in Richtung der optischen Achse 25 - in Richtung der ersten Flüssigkeit 12 gedrückt wird. Dies erfolgt in besonders einfacher und energiesparender Weise durch eine Betätigung der Membran 24.

Im Ausgangszustand befindet sich die Membran 24 in ihrer waagerechten Ausgangsstellung. Die zweite Flüssigkeit 13 weist auf der Anlagefläche 22 eine Tropfenform auf, wobei die Grenzfläche 14 zwischen den Flüssigkeiten 12 und 13 eine flache Krümmung aufweist. Dies ist durch eine durchgezogene Linie dargestellt.

Wenn nun der Elektromagnet 27 betätigt wird und das Plättchen 26 beispielsweise in Richtung des Behälterbodens 20 angezogen wird, führt dies dazu, dass sich auch die Membran 24 in Richtung des Behälterbodens 20 auslenkt. Dadurch wird die zweite Flüssigkeit 13 durch die Öffnung 28 herausgedrückt, wodurch sich die Krümmung der Grenzfläche 14 in eine wesentlich gewölbtere Form ändert, die durch eine gestrichelte Linie dargestellt ist. Die zweite Flüssigkeit 13 wird somit in Richtung der optischen Achse 25 in Richtung der ersten Flüssigkeit 12 gedrückt.

Die Ansteuerung der Mittel 23 zum Ändern der Grenzfläche kann direkt, beispielsweise über die Ciliar-Muskeln des Auges, oder aber auch manuell oder automatisch von außen erfolgen. Ebenso kann die Ansteuerung auf eine Weise erfolgen, wie sie im allgemeinen Teil der Beschreibung weiter oben erläutert worden ist.

Um eine Zerstörung des Tropfens der zweiten Flüssigkeit 13, etwa durch Erschütterungen oder dergleichen zu verhindern, sind Stabilisierungsmittel 30 vorgesehen. Bei den Stabilisierungsmitteln 30 kann es sich um eine - beispielsweise elastische - Stabilisierungsfolie 32, insbesondere eine Lochfolie, um ein Stabilisierungsnetz mit Maschenstruktur oder dergleichen handeln. Die Stabilisierungsfolie 32 ist über geeignete Befestigungselemente 33 an der Anlagefläche 22 angeordnet.

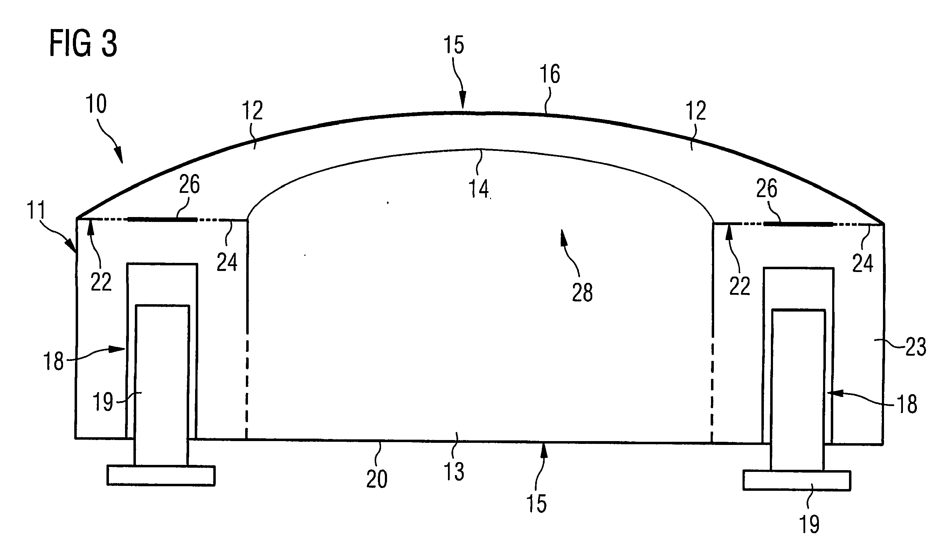

In den Figuren 3 bis 5 sind zwei andere Ausführungsbeispiele einer künstlichen Linse 10 gemäß der vorliegenden Erfindung dargestellt. Der Grundaufbau der künstlichen Linsen 10 mit Aufnahmebehälter 11 und den flexiblen Medien 12, 13 entspricht im Wesentlichen der in Figur 2 dargestellten Ausführungsform, so dass diesbezüglich zunächst auf die vorstehenden Ausführungen zu Figur 2 verwiesen wird. Dabei sind gleiche Bauteile mit identischen Bezugszeichen versehen worden. Auch bei den Ausführungsbeispielen gemäß der Figuren 3 bis 5 sind Mittel 23 zum Ändern der Grenzfläche(n) 14 zwischen den formflexiblen Medien 12, 13 vorgesehen, die mechanisch betätigbar ausgebildet sind. Selbstverständlich sind auch Ausgestaltungsformen der künstlichen Linse 10 realisierbar, die nach dem weiter oben beschriebenen Prinzip des Elektrobenetzens funktionieren.

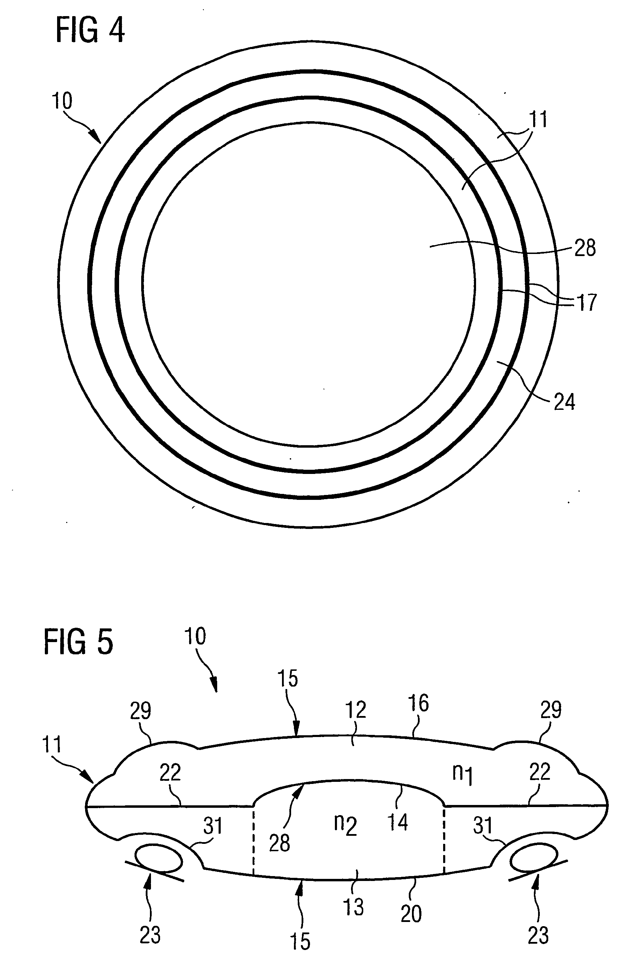

Die in den Figuren 3 und 4 dargestellte künstliche Linse 10 weist einen Aufnahmebehälter 11 mit einem Behälterboden 20 und einem Behälterdeckel 16 auf, bei denen es sich um Begrenzungen 15 des Aufnahmebehälters 11, der wiederum den Linsenkörper der künstlichen Linse 10 bildet, handelt. Der Behälterdeckel 16 weist diesmal eine gekrümmte Kontur auf und ist diesbezüglich an die Kontur der Augenlinse angepasst.

Das erste formflexible Medium 12, beispielsweise Wasser, und das zweite formflexible Medium 13, beispielsweise Öl, berühren sich gemeinsam an einer Grenzfläche 14. Es ist wiederum eine Anlagefläche 22 in Form einer Zwischenschicht vorgesehen. Die Zwischenschicht 22 verfügt über eine Öffnung 28. Die beiden Flüssigkeiten 12, 13 sind über geeignete Mittel im Aufnahmebehälter 11 räumlich fixiert, wobei die zweite Flüssigkeit 13 zumindest bereichsweise an der als Anlagefläche ausgebildeten Zwischenschicht 22 innerhalb des Aufnahmebehälters 11 anliegt. Dabei ist die zweite Flüssigkeit 13 ebenfalls im Bereich der Öffnung 28 fixiert, sodass sich die Flüssigkeit 13, die zumindest im Bereich der Grenzfläche 14 zwischen der ersten und zweiten Flüssigkeit 12, 13 eine Tropfenform aufweist, konzentrisch um die optische Achse herum erstreckt. Diesbezüglich wird zusätzlich auch auf die Ausführungen im Zusammenhang mit der Figur 2 verwiesen. Die Öffnung 28 kann beispielsweise einen Durchmesser aufweisen, der mindestens dem Pupillendurchmesser des mit der künstlichen Linse zu implantierenden Auges entspricht. Die Öffnung 28 begrenzt somit die lichte Öffnung der Linse 10.

Die Mittel 23 zum Ändern der Grenzfläche verfügen, ähnlich wie bei dem Beispiel aus Figur 2, über eine Membran 24, die diesmal jedoch kreisförmig um die Öffnung herum angeordnet ist (Figur,4). Die Membran kann Bestandteil der Zwischenschicht 22 sein und mit dem Aufnahmebehälter 11 verbunden sein. Dies kann über geeignete Befestigungsmittel 17 erfolgen. Natürlich können die Membran 24 und der Aufnahmebehälter 11 zu einem einzigen Bauteil zusammengefasst sein. Je nach Betätigungsart der Membran 24 kann in dieser wiederum wenigstens ein Plättchen 26 vorgesehen sein.

In dem Beispiel gemäß der Figuren 3 und 4 weisen die Mittel 23 zum Ändern der Grenzfläche eine Stempeleinrichtung 18 mit einem beweglichen Stempel 19 auf. Auch diese ist ringförmig um die Öffnung 28 herum angeordnet. Beispielsweise kann der Stempel 19 magnetisch sein, so dass dessen Bewegung das Plättchen 26 und damit die Membran 24 bewegen wird. Die Verschiebung der Membran 14 wirkt sich in der wie bei Figur 2 beschriebenen Weise auf die Flüssigkeiten 12, 13 aus, so dass sich die Kontur von deren Grenzfläche 14 verändert. Dadurch ändert sich auch die Brennweite der künstlichen Linse 10. Natürlich ist es auch denkbar, dass die Mittel 23 über eine Kolbeneinrichtung verfügen, wobei die Bewegung des Kolbens bereits zu einer Verdrängung der Flüssigkeit 13 führt. In anderer Ausgestaltung könnte die Bewegung der Membran auch über ein Piezoelement oder dergleichen erfolgen.

Die Ansteuerung der Mittel 23 zum Ändern der Grenzfläche kann wiederum direkt, beispielsweise über die Ciliar-Muskeln des Auges, oder aber auch manuell oder automatisch von außen erfolgen. Ebenso kann die Ansteuerung auf eine Weise erfolgen, wie sie im allgemeinen Teil der Beschreibung weiter oben erläutert worden ist.

In Figur 5 schließlich ist eine künstliche Linse 10 dargestellt, die von ihrer äußeren Erscheinungsform her bereits der Linse 102 eines Auges 100 (Figur 1) entspricht. Die Linse 10 entspricht von ihrem Grundaufbau sowie von ihrer grundsätzlichen Funktionsweise her denjenigen Linsen, die in den Figuren 2 bis 4 dargestellt sind. Gleiche Bauteile sind daher mit identischen Bezugseichen versehen. Weiterhin wird auf die entsprechenden Ausführungen zu den Figuren Bezug genommen und verwiesen.

Die künstliche Linse 10 verfügt über einen den Linsenkörper 11 bildenden Aufnahmebehälter 11, in dem sich, durch eine Trennwand 22 grundsätzlich voneinander getrennt, zwei formflexible Medien 12, 13 befinden. Bei den formflexiblen Medien 12, 13 handelt es sich wiederum um Flüssigkeiten, im Fall des Mediums 12 um Wasser, und im Fall des Mediums 13 um Öl. Die formflexiblen Medien 12, 13 weisen unterschiedliche Brechzahlen n1 und n2 auf. Die Trennwand 22 beziehungsweise die Zwischenwand weist eine Öffnung 28 auf, durch die das Medium 13 in den Bereich des Mediums 12 eintreten kann. Beide Medien 12, 13 berühren sich an einer Grenzfläche 14.

Sowohl der Behälterboden 20 als auch der Behälterdeckel 16, bei denen es sich um Begrenzungen 15 des Aufnahmebehälters 11 handelt, sind aus einem transparenten Material gebildet. Der Behälterdeckel 16 weist zumindest einen flexiblen Bereich 29 auf. Der Behälterboden 20 weist zumindest einen flexiblen Bereich 31 auf. Die flexiblen Bereiche 29 dienen zur Realisierung eines Druckausgleichs.

Im flexiblen Bereich 31 des Behälterbodens sind die Mittel 23 zum Ändern der Grenzfläche angeordnet. Dabei kann es sich beispielsweise um eine wie in den Figuren 3 und 4 dargestellte ringförmige Anordnung handeln, wobei die Erfindung selbstverständlich nicht auf diese konkrete Ausgestaltung beschränkt ist.

Durch die Betätigung der Mittel 23 zum Ändern der Grenzfläche werden die flexiblen Bereiche 31 des Behälterbodens 20 nach innen in den Aufnahmebehälter hinein gedrückt. Dadurch kommt es zu einer Verdrängung des Mediums 13, das nur durch die Öffnung 28 entweichen kann. Dies führt zu einer Veränderung der Kontur der Grenzfläche 14 zwischen den Medien 12 und 13 und damit zu einer Veränderung der Brennweite der künstlichen Linse 10. Ein entsprechender Druckausgleich im Bereich des Mediums 12 kann über die flexiblen Bereiche 29 im Behälterdeckel 16 erfolgen.

Auch hier kann die Ansteuerung der Mittel 23 zum Ändern der Grenzfläche kann direkt, beispielsweise über die Ciliar-Muskeln des Auges, oder aber auch manuell oder automatisch von außen erfolgen. Ebenso kann die Ansteuerung auf eine Weise erfolgen, wie sie im allgemeinen Teil der Beschreibung weiter oben erläutert worden ist.

Durch die vorliegende Erfindung wird eine neuartige künstliche Linse für ein Auge geschaffen. Bisher im Rahmen der Augenchirurgie in ein Auge eingesetzte künstliche Linsen wiesen immer eine feste, unveränderliche Brennweite auf. Gemäß der vorliegenden Erfindung wird nunmehr eine künstliche Linse für ein Auge bereitgestellt, bei der sich die Brennweite verändern läßt. Dies wird erfindungsgemäß dadurch realisiert, dass die künstliche Linse als Linsenelemente zwei oder mehr formflexible Medien aufweist. Die formflexiblen Medien sind vorzugsweise in einem einen Linsenkörper bildenden Aufnahmebehälter angeordnet. Dadurch werden neue Anwendungsbereiche für künstliche Linsen geschaffen, beispielsweise die Beseitigung der Alterssichtigkeit. Selbstverständlich können die erfindungsgemäßen künstlichen Linsen auch bei den bereits heute existierenden Anwendungen - etwa bei der Kataraktchirurgie - Verwendung finden.

### Bezugszeichenliste

- 10: Künstliche Linse
- 11: Aufnahmebehälter
- 12: Erstes formflexibles Medium
- 13: Zweites formflexibles Medium
- 14: Grenzfläche zwischen den Medien
- 15: Begrenzung des Aufnahmebehälters
- 16: Behälterdeckel
- 17: Befestigungsmittel
- 18: Stempeleinrichtung
- 19: Stempel
- 20: Behälterboden
- 21: Lichtstrahlrichtung
- 22: Anlagefläche (Zwischenschicht)
- 23: Mittel zum Ändern der Grenzfläche
- 24: Membran
- 25: Optische Achse
- 26: Plättchen
- 27: Elektromagnet
- 28: Öffnung
- 29: Flexibler Bereich des Behälterdeckels
- 30: Stabilisierungsmittel
- 31: Flexibler Bereich des Behälterbodens
- 32: Stabilisierungsfolie
- 33: Befestigungselement

- 100: Auge
- 101: Iris
- 102: Linse
- 103: Pupille
- 104: Zentralgefäße
- 104: Retina
- 106: Augenkammer
- 107: Glaskörperraum
- 108: Cornea

## Patentansprüche

1. Künstliche Linse (10) für ein Auge, wobei diese als Linsenelemente zwei oder mehr formflexible Medien (12, 13) aufweist, **dadurch gekennzeichnet, dass** die formflexiblen Medien (12, 13) als Flüssigkeit oder gelartig ausgebildet sind, dass die formflexiblen Medien (12, 13) in direktem Kontakt zueinander stehen, und dass sich die formflexiblen Medien (12, 13) an wenigstens einer Grenzfläche (14) berühren und gegeneinander verschiebbar angeordnet sind.

2. Künstliche Linse nach Anspruch 1, **dadurch gekennzeichnet, dass** die wenigstens zwei formflexiblen Medien (12, 13) in einem einen Linsenkörper bildenden Aufnahmebehälter (11) angeordnet sind.

3. Künstliche Linse nach Anspruch 1 oder 2, **dadurch gekennzeichnet, dass** diese zur Akkommodation geeignet ausgebildet ist.

4. Künstliche Linse nach einem der Ansprüche 1 bis 3, **dadurch gekennzeichnet, dass** diese eine definierte voreingestellte Brechkraft aufweist.

5. Künstliche Linse nach einem der Ansprüche 1 bis 4, **dadurch gekennzeichnet, dass** diese einen definierten Dynamikbereich der Brechkraft aufweist, und dass der Dynamikbereich vorzugsweise mindestens 1.5 Dioptrien, bevorzugt, mindestens 2.5 Dioptrien, ganz besonders bevorzugt mindestens 4 Dioptrien umfasst.

6. Künstliche Linse nach einem der Ansprüche 1 bis 5, **dadurch gekennzeichnet, dass** in einem einen Linsenkörper bildenden Aufnahmebehälter (11) wenigstens zwei formflexible Medien (12, 13) vorgesehen sind, und dass die Medien (12, 13) in dem Aufnahmebehälter (11) räumlich fixiert sind.

7. Künstliche Linse nach einem der Ansprüche 1 bis 6, **dadurch gekennzeichnet, dass** sich die Medien (12, 13) an wenigstens einer Grenzfläche (14) berühren und dass Mittel (23) zum Ändern der Größe und/oder Form der Grenzflächen (14) zwischen den Medien (12, 13) vorgesehen sind.

8. Künstliche Linse nach einem der Ansprüche 1 bis 7, **dadurch gekennzeichnet, dass** eine Ansteuereinrichtung zum Ansteuern der künstlichen Linse (10) vorgesehen ist.

9. Künstliche Linse nach einem der Ansprüche 1 bis 8, **dadurch gekennzeichnet, dass** die formflexiblen Medien (12, 13) nicht mischbar sind.

10. Künstliche Linse nach einem der Ansprüche 2 bis 9, **dadurch gekennzeichnet, dass** wenigstens eine Begrenzung des Aufnahmebehälters (11) eine zumindest bereichsweise gewölbte Kontur aufweist.

11. Künstliche Linse nach einem der Ansprüche 2 bis 10, **dadurch gekennzeichnet, dass** eine oder mehrere Begrenzungen des Aufnahmebehälters (11) zumindest bereichsweise transparent ausgebildet ist/sind.

12. Künstliche Linse nach einem der Ansprüche 2 bis 11, **dadurch gekennzeichnet, dass** eine oder mehrere Begrenzungen (15) des Aufnahmebehälters (11) zumindest bereichsweise aus einem flexiblen Material gebildet sind.

13. Künstliche Linse nach einem der Ansprüche 1 bis 12, **dadurch gekennzeichnet, dass** wenigstens eines der formflexiblen Medien (12, 13) zumindest bereichsweise transparent ausgebildet ist.

14. Künstliche Linse nach einem der Ansprüche 2 bis 13, **dadurch gekennzeichnet, dass** wenigstens ein formflexibles Medium (12, 13) zumindest bereichsweise an wenigstens einer Anlagefläche (22) innerhalb des Aufnahmebehälters (11) anliegt.

15. Künstliche Linse nach einem der Ansprüche 1 bis 14, **dadurch gekennzeichnet, dass** wenigstens eines der formflexiblen Medien (12, 13) in Form eines oder mehrerer Tropfen ausgebildet ist.

16. Künstliche Linse nach einem der Ansprüche 1 bis 15, **dadurch gekennzeichnet, dass** die formflexiblen Medien (12, 13) die gleiche oder annähernd die gleiche Dichte aufweisen.

17. Künstliche Linse nach Anspruch 16, **dadurch gekennzeichnet, dass** die formflexiblen Medien (12, 13) in einem bestimmten Temperaturbereich die gleiche oder annähernd die gleiche Dichte aufweisen und dass der Temperaturbereich vorzugsweise zwischen 30°C und 45°C, bevorzugt zwischen 35°C und 40°C, ganz besonders bevorzugt zwischen 36°C und 38°C liegt.

18. Künstliche Linse nach einem der Ansprüche 1 bis 17, **dadurch gekennzeichnet, dass** wenigstens zwei der formflexiblen Medien (12, 13) unterschiedliche optische Eigenschaften, insbesondere unterschiedliche Brechzahlen, aufweisen.

19. Künstliche Linse nach einem der Ansprüche 7 bis 18, **dadurch gekennzeichnet, dass** ein formflexibles Medium (13) über die Mittel (23) zum Ändern der Grenzflächen (14) derart in Richtung eines jeweils anderen formflexiblen Mediums (12) verschoben wird oder verschiebbar ist" dass sich die Krümmung wenigstens einer Grenzfläche (14) zwischen den beiden formflexiblen Medien (12, 13) ändert.

20. Künstliche Linse nach einem der Ansprüche 7 bis 19, **dadurch gekennzeichnet, dass** die Mittel (23) zum Ändern der Grenzflächen ringförmig um eine lichte Öffnung (28) herum angeordnet sind.

21. Künstliche Linse nach Anspruch 20, **dadurch gekennzeichnet, dass** die lichte Öffnung (28) zumindest dem maximalen Pupillendurchmesser des Auges entspricht, für das die künstliche Linse (10) bestimmt ist.

22. Künstliche Linse nach einem der Ansprüche 7 bis 21, **dadurch gekennzeichnet, dass** die Mittel (23) zum Ändern der Größe und/oder Form der Grenzflächen zwischen den formflexiblen Medien (12, 13) auf der Basis des Elektrobenetzens, "Electrowetting" ausgebildet sind.

23. Künstliche Linse nach Anspruch 22, **dadurch gekennzeichnet, dass** ein erstes formflexibles Medium (12) und ein zweites formflexibles Medium (13) eine unterschiedliche elektrische Leitfähigkeit aufweisen, dass das formflexible Medium (12) mit der geringeren elektrischen Leitfähigkeit zwischen dem formflexiblen Medium (13) mit der größeren elektrischen Leitfähigkeit und wenigstens einer Elektrode angeordnet ist und dass durch Anlegen eines elektrischen Feldes zwischen der wenigstens einen Elektrode und dem formflexiblen Medium (13) mit der größeren elektrischen Leitfähigkeit die Grenzfläche zwischen den beiden formflexiblen Medien (12, 13) verändert wird oder veränderbar ist..

24. Künstliche Linse nach einem der Ansprüche 7 bis 21, **dadurch gekennzeichnet, dass** die Mittel (23) zum Ändern der Grenzflächen zur Einwirkung auf wenigstens eines der formflexiblen Medien (13) ausgebildet sind, dass die Mittel (23) zum Ändern der Grenzflächen zum Erzeugen eines Drucks auf wenigstens eines der formflexiblen Medien (13) ausgebildet sind und dass ein formflexibles Medium (13) über diese Mittel (23) an wenigstens einer Grenzfläche (14) in zumindest einer Vorzugsrichtung in Richtung eines jeweils anderen formflexiblen Mediums (12) verschoben, insbesondere gedrückt, wird oder werden kann.

25. Künstliche Linse nach einem der Ansprüche 7 bis 21, **dadurch gekennzeichnet, dass** wenigstens ein formflexibles Medium allseitig von einem anderen formflexiblen Medium umschlossen ist, dass die Mittel (23) zum Ändern der Grenzflächen zur Einwirkung auf wenigstens eines der formflexiblen Medien ausgebildet sind und dass die Mittel (23) zum Ändern der Grenzflächen zum Erzeugen eines Drucks auf wenigstens eines der formflexiblen Medien ausgebildet sind.

26. Künstliche Linse nach Anspruch 24 oder 25, **dadurch gekennzeichnet, dass** die Mittel (23) zum Ändern der Grenzflächen als mechanische Mittel ausgebildet sind.

27. Künstliche Linse nach Anspruch 26, **dadurch gekennzeichnet, dass** die mechanischen Mittel (23) als Kolbeneinrichtung, Stempeleinrichtung oder Zylindereinrichtung ausgebildet sind.

28. Künstliche Linse nach einem der Ansprüche 24 bis 27, **dadurch gekennzeichnet, dass** die Mittel (23) zum Ändern der Grenzfläche(n) in Form wenigstens einer ansteuerbaren Membran (24) ausgebildet sind.

29. Künstliche Linse nach einem der Ansprüche 2 bis 28, **dadurch gekennzeichnet, dass** die räumliche Fixierung der formflexiblen Medien (12, 13) innerhalb des Aufnahmebehälters (11) über Fixiermittel erfolgt.

30. Künstliche Linse nach Anspruch 29, **dadurch gekennzeichnet, dass** die Fixiermittel in Form einer oder mehrerer unterschiedlicher Oberflächenbeschichtungen innerhalb des Aufnahmebehälters (11) und/oder in Form einer geometrischen Ausgestaltung zumindest von Bereichen des Aufnahmebehälters (11) ausgebildet sind.

31. Künstliche Linse nach einem der Ansprüche 1 bis 30, **dadurch gekennzeichnet, dass** Mittel zum Stabilisieren (Stabilisierungsmittel) der Oberflächenstruktur wenigstens eines der formflexiblen Medien (12, 13) vorgesehen sind.

32. Künstliche Linse nach Anspruch 31, **dadurch gekennzeichnet, dass** die Stabilisierungsmittel zumindest im Bereich der Grenzfläche(n) (14) zwischen zwei formflexiblen Medien (12, 13) vorgesehen sind.

## Claims

1. Artificial lens (10) for an eye, comprising as lens elements two or more flexibly shaped media (12, 13), **characterised in that** the flexibly shaped media (12, 13) are in the form of a fluid or are gel-like, **in that** the flexibly shaped media (12, 13) are in direct contact with one another, and **in that** the flexibly shaped media (12, 13) are in contact on at least one interface (14) and are arranged to be movable relative to one another.

2. Artificial lens according to claim 1, **characterised in that** the at least two flexibly shaped media (12, 13) are arranged in a receiving container (11) that forms a lens body.

3. Artificial lens according to claim 1 or 2, **characterised in that** it is constructed so as to be capable of accommodation.

4. Artificial lens according to one of claims 1 to 3, **characterised in that** it has a defined preset refractive power.

5. Artificial lens according to one of claims 1 to 4, **characterised in that** it has a defined dynamic range of refractive power, and **in that** the dynamic range preferably encompasses at least 1.5 dioptres, preferably at least 2.5 dioptres, most particularly preferably at least 4 dioptres.

6. Artificial lens according to one of claims 1 to 5, **characterised in that** at least two flexibly shaped media (12, 13) are provided in a receiving container (11) that forms a lens body and **in that** the media (12, 13) are spatially fixed in the receiving container (11).

7. Artificial lens according to one of claims 1 to 6, **characterised in that** the media (12, 13) are in contact on at least one interface (14) and **in that** means (23) are provided for changing the size and/or shape of the interfaces (14) between the media (12, 13).

8. Artificial lens according to one of claims 1 to 7, **characterised in that** an operating device is provided for operating the artificial lens (10).

9. Artificial lens according to one of claims 1 to 8, **characterised in that** the flexibly shaped media (12, 13) cannot be mixed.

10. Artificial lens according to one of claims 2 to 9, **characterised in that** at least one boundary of the receiving container (11) has a contour which is convex at least in parts.

11. Artificial lens according to one of claims 2 to 10, **characterised in that** one or more boundaries of the receiving container (11) is or are constructed to be transparent at least in parts.

12. Artificial lens according to one of claims 2 to 11, **characterised in that** one or more boundaries (15) of the receiving container (11) are formed from a flexible material, at least in parts.

13. Artificial lens according to one of claims 1 to 12, **characterised in that** at least one of the flexibly shaped media (12, 13) is constructed to be transparent at least in parts.

14. Artificial lens according to one of claims 2 to 13, **characterised in that** at least one flexibly shaped medium (12, 13) abuts, at least partially, on at least one abutment surface (22) within the receiving container (11).

15. Artificial lens according to one of claims 1 to 14, **characterised in that** at least one of the flexibly shaped media (12, 13) is constructed in the form of one or more drops.

16. Artificial lens according to one of claims 1 to 15, **characterised in that** the flexibly shaped media (12, 13) have the same or substantially the same density.

17. Artificial lens according to claim 16, **characterised in that** the flexibly shaped media (12, 13) have the same or substantially the same density in a specific temperature range and **in that** the temperature range is preferably between 30°C and 45°C, preferably between 35°C and 40°C, most particularly preferably between 36°C and 38°C.

18. Artificial lens according to one of claims 1 to 17, **characterised in that** at least two of the flexibly shaped media (12, 13) have different optical properties, particularly different refractive indices.

19. Artificial lens according to one of claims 7 to 18, **characterised in that** a flexibly shaped medium (13) is moved or movable towards another flexibly shaped medium by the means (23) for varying the interfaces (14), such that the curvature of at least one interface (14) between the two flexibly shaped media (12, 13) is altered.

20. Artificial lens according to one of claims 7 to 19, **characterised in that** the means (23) for varying the interfaces are arranged in an annular configuration around a opening (28) for light.

21. Artificial lens according to Claim 20, **characterised in that** the opening (28) for light corresponds at least to the maximum pupil diameter of the eye for which the artificial lens (10) is intended.

22. Artificial lens according to one of claims 7 to 21, **characterised in that** the means (23) for varying the size and/or shape of the interfaces between the flexibly shaped media (12, 13) are constructed according to the "electrowetting" principle.

23. Artificial lens according to claim 22, **characterised in that** a first flexibly shaped medium (12) and a second flexibly shaped medium (13) have different electrical conductivities, **in that** the flexibly shaped medium (12) with the lower electrical conductivity is arranged between the flexibly shaped medium (13) with the higher electrical conductivity and at least one electrode, and **in that** by applying an electrical field between the at least one electrode and the flexibly shaped medium (13) with the higher electrical conductivity the interface between the two flexibly shaped media (12, 13) is or can be varied.

24. Artificial lens according to one of claims 7 to 21, **characterised in that** the means (23) for varying the interfaces are designed to act on at least one of the flexibly shaped media (13), **in that** the means (23) for varying the interfaces are designed to generate pressure on at least one of the flexibly shaped media (13), and **in that**, using these means (23), a flexibly shaped medium (13) is or can be pushed, particularly pressed, on at least one interface (14) in at least one preferential direction towards another flexibly shaped medium (12).

25. Artificial lens according to one of claims 7 to 21, **characterised in that** at least one flexibly shaped medium is entirely surrounded by another flexibly shaped medium, **in that** the means (23) for varying the interfaces are designed to act on at least one of the flexibly shaped media and **in that** the means (23) for varying the interfaces are designed to generate pressure on at least one of the flexibly shaped media.

26. Artificial lens according to claim 24 or 25, **characterised in that** the means (23) for varying the interfaces are constructed as mechanical means.

27. Artificial lens according to claim 26, **characterised in that** the mechanical means (23) are constructed as a piston device, ram device or cylinder device.

28. Artificial lens according to one of claims 24 to 27, **characterised in that** the means (23) for varying the interface(s) are in the form of at least one controllable membrane (24).

29. Artificial lens according to one of claims 2 to 28, **characterised in that** the spatial fixing of the flexibly shaped media (12, 13) within the receiving container (11) is effected by fixing means.

30. Artificial lens according to claim 29, **characterised in that** the fixing means are constructed in the form of one or more different surface coatings inside the receiving container (11) and/or in the form of a geometric configuration of at least parts of the receiving container (11).

31. Artificial lens according to one of claims 1 to 30, **characterised in that** means (stabilising means) are provided for stabilising the surface structure of at least one of the flexibly shaped media (12, 13).

32. Artificial lens according to claim 31, **characterised in that** the stabilising means are provided at least in the region of the interface(s) (14) between two flexibly shaped media (12,13).

## Revendications

1. Lentille artificielle (10) pour un oeil, celle-ci comprenant comme éléments de lentille deux milieux déformables (12, 13) ou plus, **caractérisée en ce que** les milieux déformables (12, 13) sont conçus comme un fluide ou un gel, **en ce que** les milieux déformables (12, 13) sont en contact direct l'un avec l'autre, et **en ce que** les milieux déformables (12, 13) se touchent sur au moins une surface limite (14) et sont disposés de manière à pouvoir se déplacer l'un vers l'autre.

2. Lentille artificielle selon la revendication 1, **caractérisée en ce que** les au moins deux milieux déformables (12, 13) sont disposés dans un conteneur de réception (11) formant un corps de lentille.

3. Lentille artificielle selon la revendication 1 ou 2, **caractérisée en ce que** celle-ci est conçue de manière appropriée pour l'accommodation.

4. Lentille artificielle selon l'une quelconque des revendications 1 à 3, **caractérisée en ce que** celle-ci présente une réfringence préréglée définie.

5. Lentille artificielle selon l'une quelconque des revendications 1 à 4, **caractérisée en ce que** celle-ci comprend une plage de mesure définie de la réfringence, et **en ce que** la plage de mesure comporte de préférence au moins 1,5 dioptres, au moins 2,5 dioptres, de manière particulièrement préférée au moins 4 dioptres.

6. Lentille artificielle selon l'une quelconque des revendications 1 à 5, **caractérisée en ce qu'**au moins deux milieux déformables (12, 13) sont prévus dans un conteneur de réception (11) formant un corps de lentille, et **en ce que** les milieux (12, 13) sont fixés dans l'espace dans le conteneur de réception (11).

7. Lentille artificielle selon l'une quelconque des revendications 1 à 6, **caractérisée en ce que** les milieux (12, 13) se touchent sur au moins une surface limite (14) et **en ce que** des moyens (23) destinés à modifier la taille et/ou la forme des surfaces limites (14) entre les milieux (12, 13) sont prévus.

8. Lentille artificielle selon l'une quelconque des revendications 1 à 7, **caractérisée en ce qu'**il est prévu un dispositif de commande destiné à commander la lentille artificielle (10).

9. Lentille artificielle selon l'une quelconque des revendications 1 à 8, **caractérisée en ce que** les milieux déformables (12, 13) ne sont pas miscibles.

10. Lentille artificielle selon l'une quelconque des revendications 2 à 9, **caractérisée en ce qu'**au moins une délimitation du conteneur de réception (11) comprend un contour incurvé au moins par zones.

11. Lentille artificielle selon l'une quelconque des revendications 2 à 10, **caractérisée en ce qu'**un ou plusieurs délimitations du conteneur de réception (11) est/sont conçue(s) de manière transparente au moins par zones.

12. Lentille artificielle selon l'une quelconque des revendications 2 à 11, **caractérisée en ce qu'**une ou plusieurs délimitations (15) du conteneur de réception (11) sont formées à partir d'un matériau flexible au moins par zones.

13. Lentille artificielle selon l'une quelconque des revendications 1 à 12, **caractérisée en ce qu'**au moins un des milieux déformables (12, 13) est conçu de manière transparente au moins par zones.

14. Lentille artificielle selon l'une quelconque des revendications 2 à 13, **caractérisée en ce qu'**au moins un milieu déformable (12, 13) se situe au moins par zones contre au moins une surface d'appui (22) à l'intérieur du conteneur de réception (11).

15. Lentille artificielle selon l'une quelconque des revendications 1 à 14, **caractérisée en ce qu'**au moins un des milieux déformables (12, 13) est conçu sous forme d'une ou de plusieurs gouttes.

16. Lentille artificielle selon l'une quelconque des revendications 1 à 15, **caractérisée en ce que** les milieux déformables (12, 13) présentent la même ou presque la même densité.

17. Lentille artificielle selon la revendication 16, **caractérisée en ce que** les milieux déformables (12, 13) présentent dans une plage de températures définie la même ou presque la même densité et **en ce que** la plage de températures est comprise de préférence entre 30°C et 45°C, de préférence entre 35°C et 40°C, de manière particulièrement préférable entre 36°C et 38°C.

18. Lentille artificielle selon l'une quelconque des revendications 1 à 17, **caractérisée en ce qu'**au moins deux des milieux flexibles (12, 13) présentent différentes propriétés optiques, en particulier différents indices de réfraction.

19. Lentille artificielle selon l'une quelconque des revendications 7 à 18, **caractérisée en ce qu'**un milieu déformable (13) est déplacé ou peut être déplacé par les moyens (23) destinés à modifier les surfaces limites (14) en direction de respectivement un autre milieu déformable (12) de telle sorte que la courbure d'au moins une surface limite (14) entre les deux milieux déformables (12, 13) varie.

20. Lentille artificielle selon l'une quelconque des revendications 7 à 19, **caractérisée en ce que** les moyens (23) destinés à modifier les surfaces limites sont disposés de manière annulaire autour d'une ouverture intérieure (28).

21. Lentille artificielle selon la revendication 20, **caractérisée en ce que** l'ouverture intérieure (28) correspond au moins au diamètre maximal de la pupille de l'oeil pour lequel la lentille artificielle (10) est définie.

22. Lentille artificielle selon l'une quelconque des revendications 7 à 21, **caractérisée en ce que** les moyens (23) destinés à modifier la taille et/ou la forme des surfaces limites entre les milieux déformables (12, 13) sont conçus sur la base de l'électromouillage, "electrowetting".

23. Lentille artificielle selon la revendication 22, **caractérisée en ce qu'**un premier milieu déformable (12) et un second milieu déformable (13) présentent une conductivité électrique différente, **en ce que** le milieu déformable (12) à conductivité électrique plus faible est disposé entre le milieu déformable (13) à conductivité électrique plus élevée et au moins une électrode et **en ce qu'**en appliquant un champ électrique entre la au moins une électrode et le milieu déformable (13) à conductivité électrique plus élevée, la surface limite entre les deux milieux déformables (12, 13) est modifiée ou modifiable.

24. Lentille artificielle selon l'une quelconque des revendications 7 à 21, **caractérisée en ce que** les moyens (23) destinés à modifier les surfaces limites sont conçus pour agir sur au moins un des milieux déformables (13), **en ce que** les moyens (23) destinés à modifier les surfaces limites sont conçus pour produire une pression sur au moins un des milieux déformables (13) et **en ce qu'**un milieu déformable (13) est ou peut être déplacé, en particulier pressé, par ces moyens (23) contre au moins une surface limite (14) dans au moins un sens d'étirage en direction de respectivement un autre milieu déformable (12).

25. Lentille artificielle selon l'une quelconque des revendications 7 à 21, **caractérisée en ce qu'**au moins un milieu déformable est entouré de tous côtés par un autre milieu déformable, **en ce que** les moyens (23) destinés à modifier les surfaces limites sont conçus pour agir sur au moins un des milieux déformables et **en ce que** les moyens (23) destinés à modifier les surfaces limites sont conçus pour produire une pression sur au moins un des milieux déformables.

26. Lentille artificielle selon la revendication 24 ou 25, **caractérisée en ce que** les moyens (23) destinés à modifier les surfaces limites sont conçus comme des moyens mécaniques.

27. Lentille artificielle selon la revendication 26, **caractérisée en ce que** les moyens mécaniques (23) sont conçus comme un dispositif de piston, un dispositif de poinçon ou un dispositif de cylindre.

28. Lentille artificielle selon l'une quelconque des revendications 24 à 27, **caractérisée en ce que** les moyens (23) destinés à modifier la(les) surface(s) limite (s) sont conçus sous forme d'au moins une membrane commandable (24).

29. Lentille artificielle selon l'une quelconque des revendications 2 à 28, **caractérisée en ce que** la fixation dans l'espace des milieux déformables (12, 13) à l'intérieur du conteneur de réception (11) s'effectue par des moyens de fixation.

30. Lentille artificielle selon la revendication 29, **caractérisée en ce que** les moyens de fixation sont conçus sous forme d'un ou de plusieurs différents revêtements de surface à l'intérieur du conteneur de réception (11) et/ou sous forme d'une configuration géométrique au moins par zones du conteneur de réception (11).

31. Lentille artificielle selon l'une quelconque des revendications 1 à 30, **caractérisée en ce que** des moyens destinés à stabiliser (moyens de stabilisation) la structure superficielle d'au moins un des milieux déformables (12, 13) sont prévus.

32. Lentille artificielle selon la revendication 31, **caractérisée en ce que** les moyens de stabilisation sont prévus au moins dans la zone de la (des) surface(s) limite(s) (14) entre deux milieux déformables (12, 13).
